**Europäisches Patentamt**

**European Patent Office**

**Office européen des brevets**

(19)

(11) Veröffentlichungsnummer: **0 241 902**
**A1**

## (12) EUROPÄISCHE PATENTANMELDUNG

(21) Anmeldenummer: **87105491.2**

(22) Anmeldetag: **14.04.87**

(51) Int. Cl.⁴: **C 07 C 9/04, C 07 C 1/02**

(30) Priorität: **16.04.86 DE 3612734**

(43) Veröffentlichungstag der Anmeldung: **21.10.87**
**Patentblatt 87/43**

(84) Benannte Vertragsstaaten: **AT BE CH FR GB LI NL SE**

(71) Anmelder: **Kernforschungsanlage Jülich Gesellschaft mit beschränkter Haftung, Postfach 1913, D-5170 Jülich 1 (DE)**
Anmelder: **Rheinische Braunkohlenwerke AG., Stüttgenweg 2, D-5000 Köln 41 (DE)**

(72) Erfinder: **Range, Jochen, Dr., Artilleriestrasse 35, D-5170 Jülich (DE)**
Erfinder: **Höhlein, Bernd, Dr., Korbweg 4, D-5172 Linnich-Tetz (DE)**
Erfinder: **Niessen, Hans, Dr., Dampfmühlenstrasse 99, D-5160 Düren (DE)**
Erfinder: **Vau, Volker, Pfarrer-Floss-Strasse 6, D-5170 Jülich-Broich (DE)**
Erfinder: **Schiebahn, Heinrich J.R., Lyatenstrasse 3, D-5162 Niederzier 1 (DE)**
Erfinder: **Hoffmann, Horst, Broicher Strasse 60, D-5110 Alsdorf (DE)**
Erfinder: **Vorwerk, Manfred, Charles-de-Gaulle-Strasse 12, D-5140 Erkelenz (DE)**

(54) **Verfahren zur katalytischen Methanisierung eines Kohlenmonoxid, Kohlendioxid und Wasserstoff enthaltenden Synthesegases und Reaktor zur Methanisierung.**

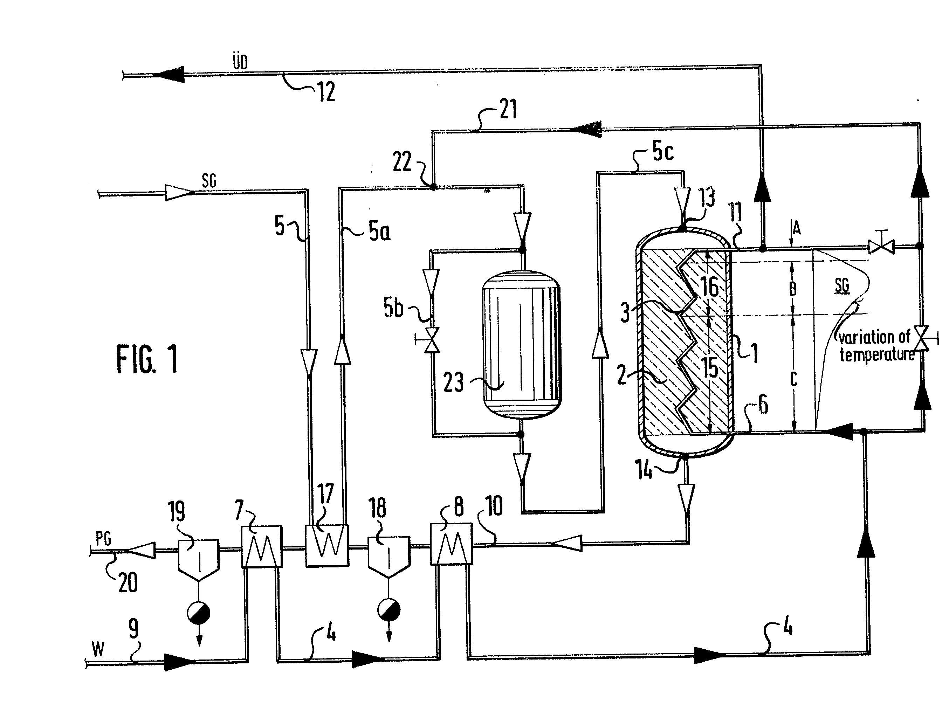

(57) Zur katalytischen Methanisierung eines Synthesegases (5), das Kohlenmonoxid, Kohlendioxid und Wasserstoff enthält, wird ein gekühltes Feststoff-Katalysatorbett (2) eingesetzt. Das Synthesegas durchströmt im Katalysatorbett nacheinander einen Eingangsbereich (A) für das Synthesegas, einen Bereich mit hoher Synthesegastemperatur (Hot-Spot-Bereich) (B) und einen Ausgangsbereich (C) mit im wesentlichen abklingender Synthesegastemperatur. Das die bei der Methanisierung entstehende Wärme aufnehmende Kühlmittel wird dabei in überhitzten Dampf (11, 12) überführt. Um das Verfahren einstufig zu führen, wird das Kühlmittel zumindest im Ausgangsbereich (C) des Katalysatorbettes (2) bei Siedetemperatur verdampft und der gebildete Dampf im Hot-Spot-Bereich (B) überhitzt. Zur Verdampfung des Kühlmittels wird neben dem Ausgangsbereich (C) auch der Hot-Spot-Bereich (B) genutzt. Ein Methanisierungsreaktor (1) zur Durchführung des Verfahrens ist in Fig. 1 dargestellt.

EP 0 241 902 A1

ACTORUM AG

Kernforschungsanlage Jülich
Gesellschaft mit beschränkter Haftung

Verfahren zur katalytischen Methanisierung eines
Kohlenmonoxid, Kohlendioxid und Wasserstoff
enthaltenden Synthesegases und Reaktor zur Methanisierung

---

Die Erfindung bezieht sich auf ein Verfahren
zur katalytischen Methanisierung eines Kohlenmonoxid, Kohlendioxid und Wasserstoff enthaltenden
Synthesegases. Die Methanisierung findet in
einem Feststoff-Katalysatorbett statt, das von
einem Kühlmittel gekühlt wird. Das Synthesegas
durchströmt im Katalysatorbett nacheinander
zunächst einen Eingangsbereich für das Synthesegas, anschließend einen Bereich mit hoher Synthesegastemperatur, der im folgenden als Hot-Spot-
Bereich bezeichnet wird, und schließlich einen
Ausgangsbereich, in dem die Synthesegastemperaturen im wesentlichen absinken. Das Kühlmittel,
das die im Feststoff-Katalysatorbett entstehende
Wärme abführt, dient der Erzeugung von Dampf
und wird in überhitzten Dampf überführt.

Die Umwandlung eines Kohlenmonoxid, Kohlendioxid und Wasserstoff enthaltenden Synthesegases
ist, da sie exotherm verläuft, zur Gewinnung

PT 1.811 G
mz/bo

- 2 -

von Energie nutzbar. Es ist bekannt, die Umwandlung der Synthesegase in Katalysatoren enthaltenden innengekühlten Reaktoren durchzuführen. Zur Dampferzeugung weisen die innengekühlten Reaktoren kühlmitteldurchströmte Kühlsysteme auf, die innerhalb des Katalysatorbettes angeordnet sind. Durch die Kühlung wird dafür Sorge getragen, daß eine für den Katalysator maximal zulässige Betriebstemperatur nicht überschritten wird, damit das Katalysatormaterial stabil bleibt.

Innengekühlte Reaktoren zur Methanisierung von Sythesegas werden in B. Höhlein, "Methanisierungsverfahren unter Berücksichtigung der Arbeiten zum NFE-Projekt", Berichte der Kernforschungsanlage Jülich, JÜL-1589, Mai 1979 beschrieben. In dieser Veröffentlichung gezeigte Anlagen, insbesondere Bild 10, erlauben eine Führung des Prozesses ohne Rückleiten von Gas mittels störanfälligen Kompressoren. Aus der DE-OS 29 49 588 ist es zur Erzeugung überhitzten Sattdampfes bekannt, mehrere Reaktoren hintereinander zu schalten, wobei einer der Reaktoren, der vom Synthesegas zuerst durchströmte Reaktor, als adiabater Reaktor, der nachfolgende Reaktor als innengekühlter Reaktor ausgebildet ist. Die im innengekühlten Reaktor entstehende Wärme wird durch Verdampfen des Kühlmittels abgeführt. Die Überhitzung des Kühlmittels erfolgt im Wärme-

0241902

austausch mit dem dem adiabaten Reaktor entströmenden Synthesegas. Hinsichtlich der Dampferzeugung im innengekühlten Reaktor und der
Überhitzung des Dampfes mit Synthesegas, das
den adiabaten Reaktor verläßt, unterscheidet
sich das aus DE-PS 31 12 991 bekannte Methanisierungsverfahren vom vorgenannten Verfahren
nicht. Von den bei diesem bekannten Verfahren
vom Synthesegas hintereinander durchströmten
drei Reaktoren wird der erste zur Erzeugung
von Dampf bei Verdampfungstemperatur, der zweite Reaktor als adiabater Reaktor, der dritte
Reaktor zur Aufheizung des Kühlmittels bis auf
Verdampfungstemperatur genutzt. Zur Überhitzung
des Kühlmittelss dient das vom adiabaten Reaktor
abströmende Synthesegas.

Abgesehen vom apparativen Aufwand mit mehreren
hintereinander zu durchströmenden Reaktoren
und der Anordnung von zusätzlichen Wärmetauschern
zur Überhitzung des erzeugten Dampfes weisen
die bekannten Verfahren auch Nachteile hinsichtlich der Verfahrensführung auf. Es ist notwendig,
um Überhitzungen der Katalysatoren zu vermeiden,
das Synthesegas zwischen den einzelnen Reaktoren
zwischenzukühlen, so daß der Methanisierungsprozeß insgesamt nur schrittweise durchführbar
ist.

Aufgabe der Erfindung ist es, zur Methanisierung von Synthesegas ein einstufiges Verfahren vorzuschlagen, das sowohl die Erzeugung überhitzten
Dampfes als auch die Erzeugung eines methanreichen
Produktgases erlaubt und gewährleistet, daß innerhalb des Katalysatorbettes auch im Hot- Spot-
Bereich eine ausreichende, die Stabilität des
Katalysators nicht beeinträchtigende Betriebstemperatur eingehalten wird.

Diese Aufgabe wird bei einem Verfahren der eingangs
erwähnten Art gemäß der Erfindung durch die in
Patentanspruch 1 angegebenen Merkmale gelöst.
Danach wird das Kühlmittel zumindest im Ausgangsbereich des Katalysatorbettes bei Siedetemperatur
verdampft, während im Hot-Spot-Bereich der gebildete Dampf überhitzt wird. Durch diese Verfahrensführung mit einer weitgehend konstanten
Temperatur des Kühlmittels im Ausgangsbereich
und der Überhitzung des gebildeten Dampfes im
Hot-Spot-Bereich läßt sich das Verfahren in einfacher Weise im optimalen Betriebsbereich des
Katalysatorbettes führen.

In weiterer Ausbildung der Erfindung gemäß Patentanspruch 2 ist vorgesehen, das Kühlmittel im
Ausgangsbereich des Katalysatorbettes im Gegenstrom
zum Synthesegas zu führen und mit einer Temperatur
in den Ausgangsbereich einzuleiten, die unterhalb
der Siedetemperatur des Kühlmittels liegt. Auf

0241902

diese Weise läßt sich am Ausgang des Katalysatorbettes auch für das Synthesegas eine niedrige
Ausgangstemperatur erreichen, so daß das Komponentengleichgewicht im Synthesegas in gewünschter
Weise zugunsten von Methan verschiebbar ist.

Eine niedrige Maximaltemperatur im Hot-Spot-Bereich läßt sich dadurch erreichen, daß der im
Ausgangsbereich gebildete Dampf im Eingangsbereich
des Katalysatorbettes eingeführt wird und im
Gleichstrom zum Synthesegas den Eingangsbereich
und den Hot-Spot-Bereich durchströmt, Patenanspruch 3. Eine weitere Erniedrigung der Maximaltemperatur ergibt sich bei einer Nutzung auch
des Hot-Spot-Bereiches zur Verdampfung des Kühlmittels, Patentanspruch 4. Für diesen zuletzt
genannten Fall dient die im Hot-Spot-Bereich
entstehende Reaktionswärme nicht nur zur Erzeugung
überhitzten Dampfes, ein Teil der Wärme wird
auch zur Verdampfung des Kühlmittels abgegeben,
was die Regelbarkeit des gewünschten Prozeßverlaufes erleichtert.

Im folgenden wird das erfindungsgemäße Verfahren
und ein Reaktor zur Durchführung dieses Verfahrens, der ebenfalls Gegenstand der Erfindung ist, anhand von Ausführungsbeispielen näher
erläutert. Es zeigen im einzelnen

0241902

Figur 1       Fließbild einer einstufigen Methanisierungsanlage;

Figur 2       Einstufige Methanisierungsanlage
              mit Dampfkammer zwischen Verdampfer
              und Überhitzer;

Figur 3       Temperaturverlauf im Katalysatorbett eines Methanisierungsreaktors
              bei Strömungsführung des Kühlmittels
              im Gegenstrom zum Synthesegas; die
              Eintrittszone wird vom Kühlmittel
              nicht durchströmt;

Figur 4       Temperaturverlauf im Katalysatorbett
              bei Strömungsführung des Kühlmittels
              im Gegenstrom zum Synthesegas mit
              einem Kühlsystem, das sich im Methanisierungsreaktor vom Ausgangsbereich
              bis zum Eingangsbereich erstreckt;

Figur 5       Temperaturverlauf im Katalysatorbett eines Methanisierungsreaktors,
              der einen vom Verdampfer getrennt
              angeordneten Überhitzer enthält,
              bei Strömungsführung des Kühlmittels
              im Gegenstrom zum Synthesegas; die
              Eintrittszone wird vom Kühlmittel
              nicht durchströmt;

Figur 6       Temperaturverlauf im Katalysatorbett eines Methanisierungsreaktors
              mit getrennt angeordnetem Verdampfer
              .und Überhitzer und zusätzlicher Kühlung
              des Eingangsbereiches des Methanisierungsreaktors (der Eingangsbereich
              dient zur Kühlmittelveränderung);

Figur 7      Temperaturverlauf im Katalysatorbett eines Methanisierungsreaktors
             mit getrennt angeordnetem Verdampfer
             und Überhitzer, wobei der Überhitzer
             vom zu überhitzenden Dampf im Gleichstrom zum Synthesegas durchströmt
             wird;

Figur 8      Temperaturverlauf im Katalysatorbett bei Verdampfung des Kühlmittels
             auch im Hot-Spot- und Eingangsbereich
             des Mathanisierungsreaktors.


Figur 1 zeigt eine Methanisierungsanlage mit
einem Methanisierungsreaktor 1, der ein Katalysatorbett 2 enthält und von einem Kühlsystem 3
durchzogen ist. Das Kühlmittel durchströmt das
Kühlsystem 3 im Gegenstrom zum Synthesegas.
Die Strömungsrichtung des Kühlmittels in Kühlmittelleitungen 4 ist in der Zeichnung jeweils
mit dunkel ausgefüllten Pfeilen, die Strömungsrichtung des Synthesegases in Synthesegasleitungen
5 mit hellen Pfeilen markiert. Als Kühlmittel
dient Wasser, das vor Eintritt in den Methanisierungsreaktor an dessen Kühlmitteleingang 6 zunächst zwei Vorwärmer 7, 8 passiert. In den
Vorwärmern wird das über den Zufluß 9 mit Raumtemperatur einströmende Wasser im Wärmeaustausch
mit Produktgas, das vom Methanisierungsreaktor
in Produktgasleitungen 10 abströmt, auf die
erforderliche Eingangstemperatur am Kühlmitteleingang 6 vorgewärmt. Das Wasser wird dann im Kühlsystem 3 des Methanisierungsreaktors durch
Aufnahme der im Katalysatorbett 2 bei der Methanisierung des Synthesegases entstehenden Wärme
verdampft und anschließend überhitzt. Der überhitzte Dampf strömt vom Kühlmittelausgang 11
aus dem Methanisierungsreaktor in eine Dampfleitung 12 ab.

In Strömungsrichtung des Synthesegases gesehen
weist der Methanisierungsreaktor 1 drei Zonen
mit charakteristischem Temperaturverlauf im
Katalysatorbett 2 auf. Entsprechend diesem Temperaturprofil im Katalysatorbett läßt sich der
Methanisierungsreaktor bei stabilem Betriebsverhalten in einen Eingangsbereich A mit steil
ansteigender Temperatur, in einen Hot-Spot-Bereich
B mit einem Temperaturmaximum im Katalysatorbett
und einen Ausgangsbereich C mit stetig fallender
Temperatur einteilen. Diese drei Zonen und ihr
charakteristischer Temperaturverlauf in Strömungsrichtung des Synthesegases gesehen sind
in Figur 1 über der Länge des Katalysatorbettes
schematisch dargestellt. Der Eingangsbereich
A ist dabei gekennzeichnet durch ansteigende
Synthesegastemperaturen von ca. $250^{o}C$ bis auf
etwa $450^{o}C$, der Hot-Spot-Bereich durch Synthesegastemperaturen zwischen $450^{o}C$ und $750^{o}C$ und
der Ausgangsbereich durch abfallende Temperaturen
von ca. $450^{o}C$ bis etwa $250^{o}C$.

Im Ausführungsbeispiel nach Figur 1 durchdringt
das Kühlsystem 3 in Strömungsrichtung des Kühlmittels gesehen sowohl den Ausgangsbereich C,
den Hot-Spot-Bereich B und den Eingangsbereich
A des Methanisierungsreaktors. Das Synthesegas
strömt im Methanisierungsreaktor im Gegenstrom
zum Kühlmittel. Es wird am Synthesegaseingang
13 in den Methanisierungsreaktor eingeführt
und im Eingangsbereich A bei einsetzender exothermer Methanisierungsreaktion im Katalysatorbett
2 zunächst stark erhitzt. Im Hot-Spot-Bereich B
erreicht das Synthesegas seine maximale Temperatur. Es wird zu Produktgas umgesetzt und verläßt
den Methanisierungsreaktor nach Abkühlung und
weiterer Umsetzung im Ausgangsbereich C als
methanreiches Produktgas. Das Produktgas strömt
vom Produktgasausgang 14 aus dem Methanisierungsreaktor in die Produktgasleitung 10 ab.

Zur Kühlung des Ausgangsbereiches C des Methanisierungsreaktors wird die dort vorhandene Wärme
aus dem Katalysatorbett durch Verdampfen des
Kühlmittels im Kühlsystem 3 abgeführt. Im Hot-
Spot-Bereich B wird die entstehende Wärme dem
Katalysatorbett durch Überhitzen des gebildeten
Dampfes entzogen. Das Kühlsystem 3 wirkt somit
im Ausgangsbereich C als Verdampfer 15 für das
Kühlmittel, im Hot-Spot-Bereich B als Überhitzer
16 für den im Verdampfer produzierten Kühlmitteldampf.

Verdampfer 15 und Überhitzer 16 gehen bei dem
in Figur 1 gezeigten Methanisierungsreaktor unmittelbar ineinander über. Es besteht daher
im Kühlsystem 3 keine feste Begrenzung zwischen
Verdampfer und Überhitzer. Wesentlich ist jedoch,
daß innerhalb des Teils des Kühlsystems 3, der
als Überhitzer 16 wirkt, die im Hot-Spot-Bereich
B entstehende Reaktionswärme abgeführt wird
und zur Überhitzung des im Verdampfer 15 des
Kühlsystem 3 gebildeten Dampfes dient. Im Überhitzer 16 steigt somit die Temperatur des Kühlmittels an. Sie fällt beim Ausführungsbeispiel
nach Figur 1, bei dem das Kühlsystem auch den
Eingangsbereich A durchzieht, lediglich in diesem
Bereich zum Kühlmittelausgang 11 hin leicht
ab. Im Verdampfer 15 befindet sich das Kühlmittel
hingegen auf konstanter Temperatur, nämlich
auf der Verdampfungstemperatur, die seinem Druck
entspricht. Beim Kühlsystem 3 nach Figur 1 lassen
sich daher Verdampfer 15 und Überhitzer 16 durch
ihren Temperaturverlauf für das Kühlmittel voneinander unterscheiden.

Das vom Produktgasausgang 14 abströmende Produktgas wird in der Produktgasleitung 10 zu den
Vorwärmern 7 und 8 geführt und tauscht hier
einen Teil seiner Wärme - wie bereits erwähnt -
mit dem in die Methanisierungsanlage einströmenden
Kühlmittel aus. Zusätzlich zu diesen Vorwärmern
7, 8 sind in die Produktgasleitung 10 noch ein
Vorwärmer 17 für das Synthesegas vorgesehen,
das in die Methanisierungsanlage einströmt,

0241902

sowie Flüssigkeitsabscheider 18, 19 zur Abscheidung von Wasser aus dem Produktgas eingesetzt.
Das trockene methanreiche Produktgas zieht am
Gasaustritt 20 aus der Methanisierungsanlage
ab.

Der im Methanisierungsreaktor 1 gebildete überhitzte Dampf wird im Ausführungsbeispiel nach
Figur 1 über die Dampfleitung 12 als Arbeitsmittel
entnommen und beispielsweise Dampfturbinen zur
Erzeugung elektrischer Energie zugeführt. Zu
einem Teil kann der überhitzte Dampf nach Vermischen mit vorgewärmtem Kühlmittel, das mittels
einer Zuleitung 21' aus der Kühlmittelleitung 4
entnommen wird, als Sattdampf über eine Entnahmeleitung 21 in die Synthesegasleitung 5 in das
hier dem Methanisierungsreaktor über den Vorwärmer
17 zuströmende Synthesegas eingeleitet werden.
Im Ausführungsbeispiel befindet sich hierzu
für die Entnahmeleitung 21 ein Anschluß 22 im
Teil 5a der Synthesegasleitung 5. Nach Zumischen
des Wasserdampfes kann das Synthesegas ganz
oder teilweise in einen Konvertierungsreaktor 23
eingeführt werden, um das im Synthesegas enthaltene Kohlenmonoxid teilweise zu Kohlendioxid
umzusetzen. Der Anteil nicht zu konvertierenden
Synthesegases strömt über einen regulierbaren
Bypaß 5b parallel zum Konvertierungsreaktor
23 zusammen mit dem konvertierten Teil des Synthesegases in den Methanisierungsreaktor 1 ein.

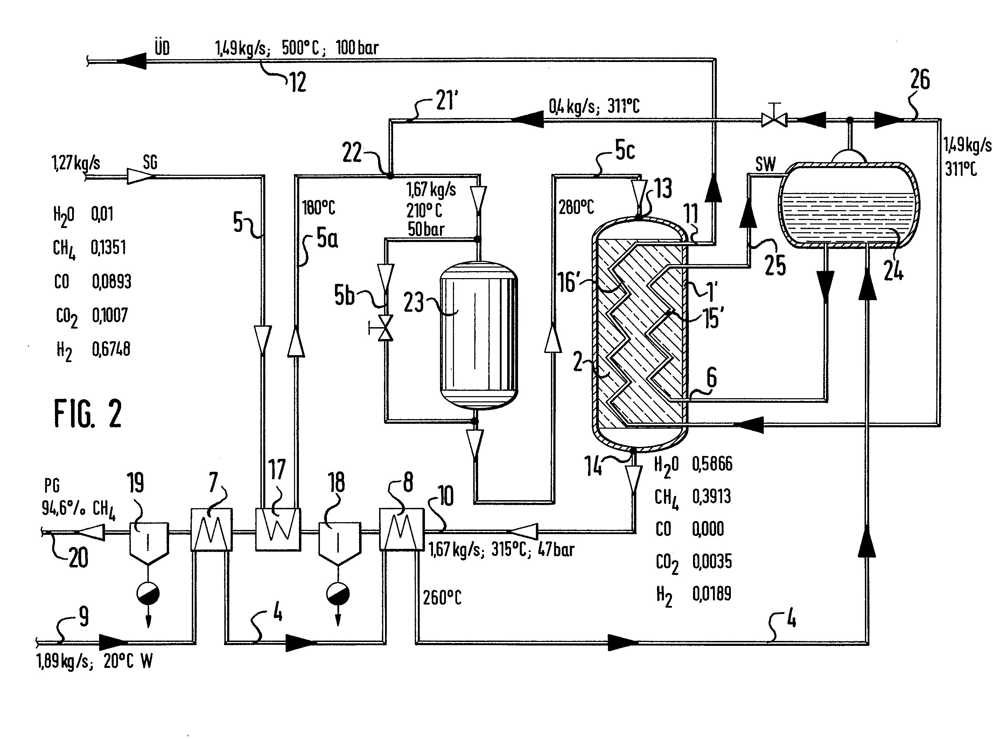

Anders als die Methanisierungsanlage nach Figur 1 weist der Methanisierungsreaktor 1' nach Figur 2 einen Verdampfer 15' und eine Überhitzer 16' auf, die im Methanisierungsreaktor voneinander getrennte Rohrsystem bilden. Zwischen Verdampfer 15' und Überhitzer 16' ist eine Dampfkammer 24 geschaltet, in der sich der vom Verdampfer 15' über eine Verbindungsleitung 25 einströmende Dampf sammelt und mitgerissene noch unverdampfte Kühlflüsssigkeit abgeschieden wird. An der höchsten Stelle der Dampfkammer 24 ist eine Dampfzuleitung 26 angeschlossen, die den sich hier sammelnden trockenen Dampf zum Überhitzer 16' leitet. Von der Dampfkammer 24 führt in gleicher Weise wie beim Ausführungsbeispiel nach Figur 1 auch eine Entnahmeleitung 21' zum Anschluß 22 im Teil 5a der Synthesegasleitung 5. Es wird somit ebenso wie im Ausführungsbeispiel nach Figur 1 Sattdampf in das Synthesegas eingeführt.

Beim Ausführungsbeispiel ist die Dampfkammer 24 außerhalb des Methanisierungsreaktors 1 angeordnet. Abweichend hiervon ist es auch möglich, die Dampfkammer innerhalb des Methanisierungsreaktors als integrierter Bestandteil einzusetzen. Die Dampfkammer wird in diesem Fall zweckmäßig oberhalb des Katalysatorbettes angeordnet und vom Synthesegas umströmt, das in den Methanisierungsreaktor eintritt.

Der im Überhitzer 16' überhitzte Dampf strömt
auch bei der Methanisierungsanlage nach Figur
2 als Arbeitsmittel z.B. zu einer Dampfturbine.
In Figur 2 sind im übrigen für alle Anlagenteile,
die unverändert den Anlagenteilen der Figur
1 entsprechen, gleiche Bezugszeichen wie in
Figur 1 angegeben.

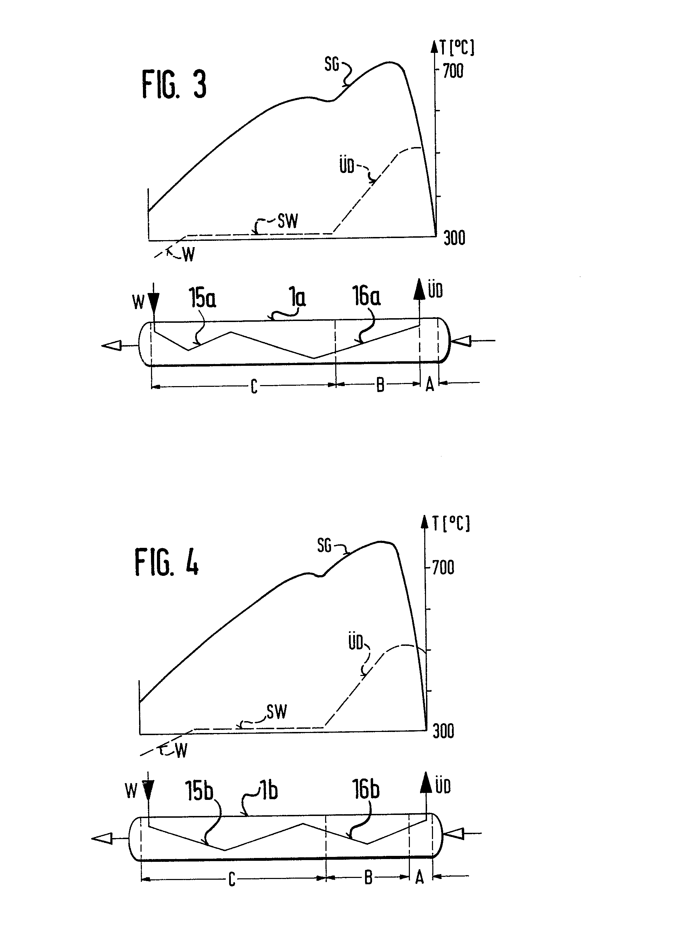

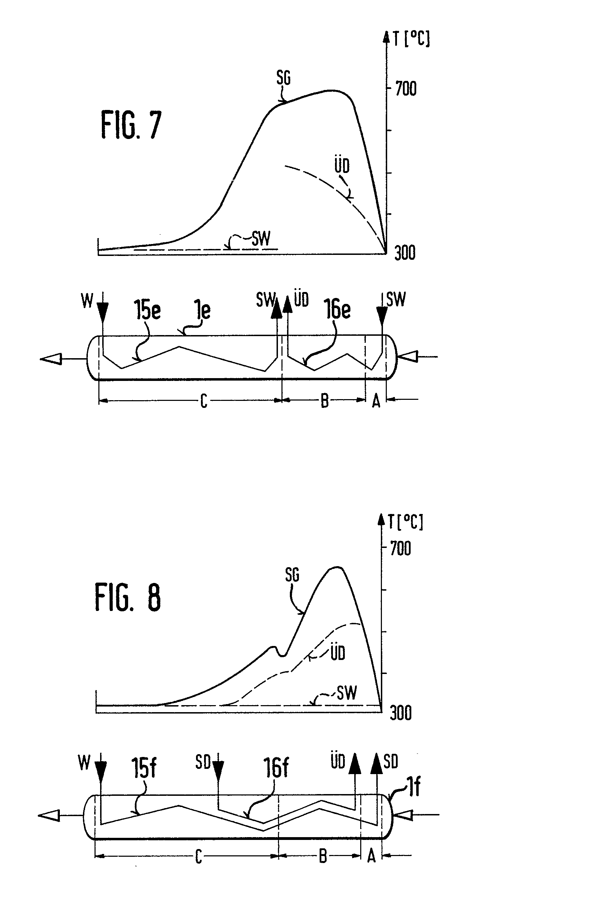

In Figuren 1 und 2 sind Verdampfer 15, 15' und
Überhitzer 16, 16' nur schematisch dargestellt.
Sie lassen sich im Katalysatorbett auch in anderer
Weise verlegen, als dies in Figuren 1 und 2
gezeigt ist. Stets befindet sich jedoch ein
Verdampfer im Ausgangsbereich C des Methanisierungsreaktors und ein Überhitzer im Hot-Spot-Bereich B. Spezielle Ausführungsformen für die
Anordnung von Verdampfer und Überhitzer sind
in Figuren 3 bis 8 wiedergegeben. Diese Figuren
zeigen auch die Temperaturverläufe, die sich
bei entsprechender Wärmeabfuhr durch das Kühlmittel im Synthesegas bzw. im Katalysatorbett 2
einstellen. Die Temperaturen sind sowohl für
das Synthesegas als auch für das Kühlmittel
jeweils über der Reaktorlänge in Strömungsrichtung
des Synthesegases bzw. des Kühlmittels eingetragen. Der Temperaturverlauf für das Synthesegas
über der Reaktorlänge ist in den Figuren mit
durchgezogenem Linienzug, der Temperaturverlauf
für das Kühlmittel mit gestricheltem Linienzug
angegeben. In allen Ausführungsbeispielen strömt das Synthesegas durch den Methanisierungsreaktor 1 vom Synthesegaseingang 13 zum Produktgasausgang 14. Die Strömungsrichtung des Kühlmittels ist im Verdampfer stets entgegengesetzt zur Strömungsrichtung des Synthesegases gerichtet. Im Überhitzer kann der Kühlmitteldampf auch im Gleichstrom zum Synthesegas geführt sein, wie dies im Ausführungsbeispiel nach Figur 7 dargestellt ist. Die Strömungsrichtung für Kühlmittel bzw. Kühlmitteldampf in Verdampfer und Überhitzer und die Strömungsrichtung für das Synthesegas im Katalysatorbett sind in Figuren 3 bis 8 in gleicher Weise wie in Figuren 1 und 2 jeweils durch dunkel ausgeführte Strömungspfeile (Kühlmittel) bzw. helle Strömungspfeile (Synthesegas) markiert.

Figur 3 zeigt einen Methanisierungsreaktor 1a, bei dem ein Kühlsystem 3a das Katalysatorbett 2 vom Ausgangsbereich C bis zum Hot-Spot-Bereich B durchzieht. Im Ausgangsbereich C dient das Kühlsystem 3a als Verdampfer 15a, im Hot-Spot-Bereich als Überhitzer 16a. Das Kühlwasser strömt in den Verdampfer 15a mit einer Temperatur unterhalb Verdampfungstemperatur ein, wird im Verdampfer auf Verdampfungstemperatur gebracht, verdampft und wird anschließend im Überhitzer 16a überhitzt. Dabei nimmt das Kühlwasser im Ausgangsbereich C, der Dampf im Hot-Spot-Bereich B die entstehende Reaktionswärme auf. Im Hot-Spot-Bereich wird der Dampf bei 100 bar bis auf eine Temperatur von ca. 500°C erhitzt. Das Katalysatorbett bleibt dabei unterhalb der adiabaten Temperatur, die bei dem im Ausführungsbeispiel verwendeten Synthesegas unterhalb 800°C liegt. Bei Übergang vom Hot-Spot-Bereich B zum Ausgangsbereich C tritt wegen der vom Verdampfer bei konstanter Siedetemperatur aufgenommenen Wärme vorallem konstruktionsbedingt, insbesondere bei Ausführung des Methanisierungsreaktors in der in DE-OS 32 47 821 beschriebenen Art, eine Unstetigkeitsstelle auf. Die Temperatur nimmt nochmals leicht zu und fällt dann auf Werte unter 400°C.

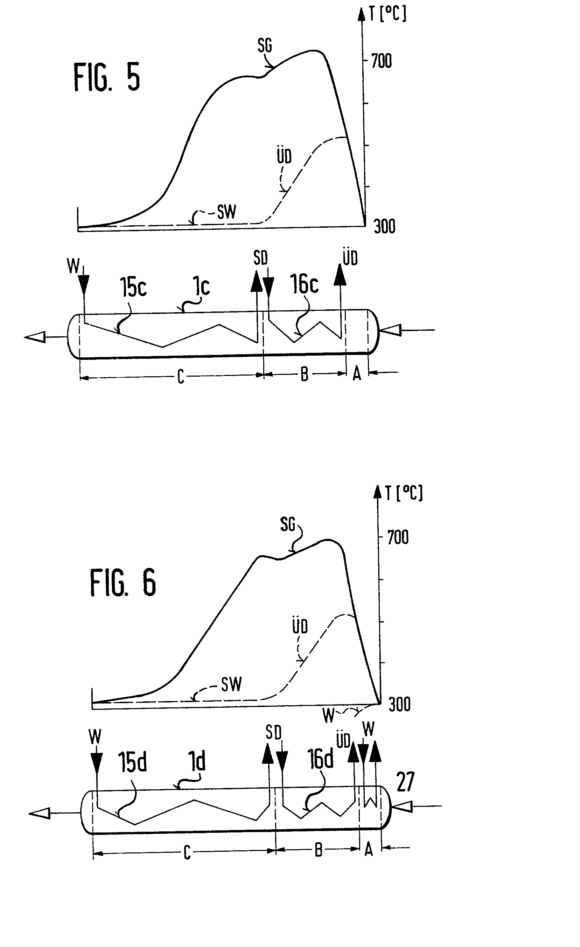

In Figur 4 weist der Methanisierungsreaktor 1b ein Kühlsystem 3b auf, das den gesamten Methanisierungsreaktor von seinem Ausgangsbereich C über den Hot-Spot-Bereich B bis zum Eingangsbereich A durchzieht. Das Kühlsystem 3b entspricht somit dem in Figur 1 dargestellten Kühlsystem 3. Am zugehörigen Temperaturverlauf in Figur 4 erkennt man, daß sich prinzipiell ein mit dem Methanisierungsreaktor nach Figur 3 vergleichbarer Temperaturverlauf einstellt. Der überhitzte Dampf wird im Eingangsbereich C jedoch vom einströmenden Synthesegas etwas unter seine Maximaltemperatur von 500°C abgekühlt. Auffällig ist im Vergleich zum Ausführungsbeispiel nach Figur 3 jedoch die starke Überhöhung der Maximaltemperatur des Synthesegases im Hot-Spot-Bereich.
Das Synthesegas wird im Katalysatorbett 2 bis
auf eine Temperatur von etwa 770°C erhitzt.
Die Ausgangstemperatur des Produktgases ist
somit auch leicht erhöht gegenüber der Ausgangstemperatur des Produktgases beim Ausführungsbeispiel nach Figur 3, sie liegt jedoch noch unterhalb 400°C.

Im Ausführungsbeispiel nach Figur 5 weist der
Methanisierungsreaktor 1c voneinander getrennte
Rohrleitungssysteme für Verdampfer 15c und Überhitzer 16c auf. Der Verdampfer 14c ist ausschließlich im Ausgangsbereich C des Methanisierungsreaktors verlegt, der Hot-Spot-Bereich B wird nur
durch Dampüberhitzung gekühlt. Das Kühlmittel
tritt in den Verdampfer bei diesem und in den
folgenden Ausführungsbeispielen bereits mit
Verdampfungstemperatur ein. Im Ausführungsbeispiel
nach Figur 5 stellen sich im Hot-Spot-Bereich
etwa die gleichen Temperaturen ein, wie im Ausführungsbeispiel nach Figur 3. Das Synthesegas
wird im Methanisierungsreaktor maximal auf ca.
730°C aufgeheizt. Es wird jedoch eine stärkere
Abkühlung des Produktgases erreicht, das Produktgas zieht mit einer Temperatur von etwa 310°C
aus dem Methanisierungsreaktor ab.

Der in Figur 6 gezeigte Methanisierungsreaktor
1d ist hinsichtlich Verdampfer 15d und Überhitzer 16d in gleicher Weise ausgerüstet wie der Methanisierungsreaktor 1c nach Figur 5. Zusätzlich weist er jedoch im Eingangsbereich einen Kühlmittelvorwärmer 27 auf. Im Kühlmittelvorwärmer wird das Kühlmittel vor Eintritt in den Verdampfer 15d auf Verdampfungstemperatur gebracht und strömt erst im Anschluß daran zum Kühlmitteleingang 6 des Verdampfers. Das Kühlmittel wird im Kühlmittelvorwärmer im Gegenstrom zum Synthesegas geführt. Durch diese Abkühlung des Synthesegases bereits im Eingangsbereich A wird die maximale Erwärmung des Katalysatorbettes im Methanisierungsreaktor auf ca. 700°C begrenzt.

700°C als Maximaltemperatur im Hot-Spot-Bereich lassen sich auch beim Ausführungsbeispiel nach Figur 7 erreichen. Bei diesem Ausführungsbeispiel wird der zu überhitzende Dampf im Überhitzer 16e im Gleichstrom zum Synthesegas geführt. der Überhitzer 16e ist im Hot-Spot-Bereich und im Eingangsbereich des Methanisierungsreaktors 1e angeordnet. Dem Eingangsbereich wird der zu überhitzende Dampf mit Siedetemperatur zugeführt. Auch bei diesem Methanisierungsreaktor wird der Dampf auf eine Temperatur von etwa 500°C bei 100 bar erhitzt. Das Produktgas strömt mit einer Temperatur von etwa 310°C aus dem Methanisierungsreaktor ab.

Eine sehr starke Erniedrigung des Temperaturmaximums im Hot-Spot-Bereich wird durch die Ausbildung
des Kühlsystems gemäß Figur 8 erreicht. Bei
diesem Ausführungsbeispiel erstreckt sich der
Verdampfer 15f des Kühlsystems über die gesamte
Länge des Katalysatorbettes vom Ausgangsbereich
C über den Hot-Spot-Bereich B bis zum Eingangsbereich A des Methanisierungsreaktors 1f. Der
Überhitzer 16f ist im Hot-Spot-Bereich angeordnet,
er ragt jedoch auch in den Ausgangsbereich C
des Methanisierungsreaktors hinein, so daß sich
Überhitzer und Verdampfer überlagern. Konstruktiv
läßt sich die in diesem Falle notwendige Wärmeübertragung zugleich auf Überhitzer 16f und
Verdampfer 15f zweckmäßig dadurch erreichen,
daß die Dampfüberhitzungsleitungen innerhalb
der die Katalysatorteilchen enthaltenden Katalysatorrohre verlaufen, so daß die Reaktionswärme
sowohl an das zu verdampfende Kühlmittel, das
die Katalysatorrohr umgibt, als auch an den
zu überhitzenden Dampf abgebbar ist. Bei dieser
Ausführung läßt sich die Maximaltemperatur des
Synthesegases bis auf $650^{o}C$ herabsetzen, ohne
daß sich die Qualität des Arbeitsmittels, nämlich
bis auf $500^{o}C$ bei 100 bar überhitzter Dampf
verschlechtert und ohne Verringerung des Methangehaltes im Produktgas. Methanisierungsreaktoren
dieser Art sind daher optimal zur Durchführung
von Methanisierungsreaktion geeignet.

In Figur 2 sind für den zuletzt genannten Fall der Ausbildung des Kühlsystems nach Figur 8 der Massenfluß, die Gasanteile in Synthesegas und Produktgas, die Temperaturen und Drücke eingetragen. Im Ausführungsbeispiel werden in die Methanisierungsanlage 1,27 kg/s Synthesegas eingeführt, das folgende Gasanteile in Vol% enthält: 1 % $H_2O$; 13,51 % $CH_4$; 8,93 % CO; 10,07 % $CO_2$; 67,48 % $H_2$. Das Synthesegas wird im Vorwärmer 17 aus etwa 180°C erhitzt und im Teil 5a der Synthesegasleitung 5 mit Sattdampf vermischt. Mit einer Temperatur von 210°C bei einem Druck von 50 bar wird im Ausführungsbeispiel bei geschlossenem Bypass 5b das gesamte Synthesegas konvertiert und dabei weiter erwärmt. Es strömt mit einer Temperatur von 280°C an Synthesegaseingang 13 in den Methanisierungsreaktor 1' ein.

Nach Umsetzung des Synthesegases im Methanisierungsreaktor erhält man am Produktgasausgang 14 ein Produktgas, das in Vol% 58,66 % $H_2O$; 39,13 % $CH_4$; 0 % CO; 0,35 % $CO_2$ und 1,89 % $H_2$ enthält. Das Produktgas strömt aus dem Methanisierungsreaktor bei einem Druck von 47 bar mit einer Temperatur von 350°C ab. Der Massenstrom des Produktgases in der Produktgasleitung beträgt 1,67 kg/s. Nach Durchströmen der Vorwärmer 8,

17 und 7 und nach Abscheidung von Wasser in den Flüssigkeitsabscheidern 18 und 19 erhält man am Gasaustritt 20 ein Produktgas mit einem Anteil von 94,6 % $CH_4$. Zum Abführen der in der Methanisierungsanlage entstehenden Wärme wird der Kühlmittelleitung 1,89 kg/s Kühlwasser mit einer Temperatur von ca. 20°C zugeführt. Das Kühlwasser wird in den Vorwärmern 7 und 8 bis auf eine Temperatur von 260°C erwärmt, mit der es im Ausführungsbeispiel in die Dampfkammer 24 eingeführt wird. In der Dampfkammer wird das Kühlwasser auf eine Temperatur von 310°C bei 100 bar gebracht und in den Verdampfer 15' eingeführt, in dem es bei einer Verdampfungstemperatur von 311°C verdampft. Der gebildete Dampf wird in die Dampfkammer 24 zurückgeführt und strömt über die Verbindungsleitung 25 in den Überhitzer 16' ein. Hier wird der Dampf auf 500°C bei 100 bar überhitzt. Es wird aus der Methanisierungsanlage 1,49 kg/s überhitzter Dampf abgeführt. Der restliche Dampf von 0,4 kg/s strömt von der Dampfkammer 24 über die Entnahmeleitung 21' am Anschluß 22 in das Synthesegas ein.

Die in Figur 2 für Synthesegas und Kühlmittel eingetragenen Daten gelten mit geringen Abweichungen auch für die Methanisierungsanlage gemäß Figur 1. Sie sind als Daten außerhalb des Methanisierungsreaktors auch zutreffend für die Ausführungsbeispiele nach Figur 3 bis 7. Zu berücksichtigen sind dabei lediglich die abweichenden
Temperaturen für das Produktgas in den Ausführungsbeispielen nach Figuren 3 und 4 sowie die
beim Ausführungsbeispiel nach Figur 4 gegenüber
allen anderen Ausführungsbeispielen unter $500^{o}C$
bleibende Temperatur für den vom Überhitzer
abströmenden überhitzten Dampf. Die in Figuren
3 bis 7 wiedergegebenen Temperaturverläufe sind
in soweit quantitativ, es handelt sich also
um halbquantitative Kurvenbilder.

0241902

Kernforschungsanlage Jülich
Gesellschaft mit beschränkter Haftung


P a t e n t a n s p r ü c h e


1. Verfahren zur katalytischen Methanisierung
   eines Kohlenmonoxid, Kohlendioxid und Wasser
   stoff enthaltenden Synthesegases in einem
   Feststoff-Katalysatorbett, das von einem
   Kühlmittel gekühlt wird, wobei das Synthesegas im Katalysatorbett nacheinander einen
   Eingangsbereich für das Synthesegas, einen
   Bereich mit hoher Synthesegastemperatur
   (Hot-Spot-Bereich) und einen Ausgangsbereich
   mit im wesentlichen abklingender Synthesegastemperatur durchströmt und wobei das
   Kühlmittel durch Übertragung der bei der
   Methanisierung entstehenden Wärme in überhitzten Dampf überführt wird, d a d u r c h
   g e k e n n z e i c h n e t , daß das Kühlmittel zumindest im Ausgangsbereich des
   Katalysatorbettes bei Siedetemperatur verdampft und der gebildete Dampf im Hot-
   Spot-Bereich überhitzt wird.


2. Verfahren nach Anspruch 1, d a d u r c h
   g e k e n n z e i c h n e t , daß das
   Kühlmittel im Ausgangsbereich im Gegenstrom
   zum Synthesegas geführt und in den Ausgangsbereich mit einer Temperatur unterhalb
   Siedetemperatur eingeleitet wird.


- 2 -

3. Verfahren nach Anspruch 1 oder 2, d a -
   d u r c h   g e k e n n z e i c h n e t ,
   daß der im Ausgangsbereich gebildete Dampf
   zur Überhitzung in den Eingangsbereich
   des Katalysatorbettes eingeführt wird und
   den Eingangsbereich und den Hot-Spot-Bereich
   im Gleichstrom zum Synthesegas durchströmt.

4. Verfahren nach Anspruch 1, 2 oder 3, d a -
   d u r c h   g e k e n n z e i c h n e t ,
   daß zur Verdampfung des Kühlmittels neben
   dem Ausgangsbereich auch der Hot-Spot-Bereich
   genutzt wird.

5. Methanisierungsreaktor zur Durchführung
   des Verfahrens nach Anspruch 1 mit einem
   Zugang für zur Methanisierung geeignetem
   Synthesegas, das Kohlenmonoxid, Kohlendioxid
   und Wasserstoff enthält, mit einem vom
   Synthesegas durchströmbaren Feststoff-Katalysatorbett und einem Ausgang für methanisiertes Produktgas und mit einem das Katalysatorbett kühlenden Kühlsystem, wobei der
   Katalysatorraum in Strömungsrichtung des
   Synthesegases gesehen einen Eingangsbereich
   für Synthesegas, einen Bereich mit hoher
   Synthesegastemperatur (Hot-Spot-Bereich)
   und einen Ausgangsbereich mit abklingender
   Synthesegastemperatur aufweist, d a -
   d u r c h   g e k e n n z e i c h n e t ,
   daß das Kühlsystem (3) im Ausgangsbereich-

0241902

(C) zur Verdampfung des Kühlmittels als
Verdampfer (15, 15'), im Hot-Spot-Bereich
(B) zur Überhitzung des entstandenen Kühlmitteldampfes als Überhitzer (16, 16')
dient.

6. Methanisierungsreaktor nach Anspruch 5,
   d a d u r c h  g e k e n n z e i c h n e t ,
   daß Verdampfer (15') und Überhitzer (16')
   im Katalysatorbett (2) voneinander getrennte
   Leitungssysteme bilden.

7. Methanisierungsreaktor nach Anspruch 6,
   d a d u r c h  g e k e n n z e i c h -
   n e t ,  daß der Verdampfer (15f) neben
   dem Ausgangsbereich (C) zumindest teilweise
   auch den Hot-Spot-Bereich (B) durchzieht.

8. Methanisierungsreaktor nach Anspruch 6
   oder 7, d a d u r c h  g e k e n n -
   z e i c h n e t ,  daß dem Verdampfer
   (15') eine Dampfkammer (24) nachgeschaltet
   ist, an der eine zum Überhitzer (16') führende Dampfzuleitung (26) angeschlossen
   ist.

9. Methanisierungsreaktor nach einem der vorhergehenden Ansprüche 5 bis 8, d a d u r c h
   g e k e n n z e i c h n e t ,  daß zum Einleiten von Dampf in eine Synthesegasleitung
   (5, 5a), die zum Synthesegaseingang (13)
   des Methanisierungsreaktors (1, 1') führt, mit der Synthesegasleitung (5, 5a) eine
Entnahmeleitung (21, 21') für Dampf verbunden
ist.

10. Methanisierungsreaktor nach Anspruch 9,
    d a d u r c h   g e k e n n z e i c h n e t ,
    daß in der Synthesegasleitung (5) zwischen
    Anschluß (22) der Entnahmeleitung (21,
    21') und Synthesegaseingang (13) am Methani-
    sierungsreaktor (1, 1') ein Konvertierungs-
    reaktor (23) angeordnet ist.

11. Methanisierungsreaktor nach Anspruch 10,
    d a d u r c h   g e k e n n z e i c h -
    n e t ,   daß parallel zum Konvertierungs-
    reaktor (23) ein regulierbarer Bypaß (5b)
    geführt ist.

FIG. 1

FIG. 2

ÜD    1,49 kg/s;  500°C;  100 bar    12

21'    0,4 kg/s; 311°C    26

1,49 kg/s 311°C

22

1,27 kg/s    SG    5    5a    180°C    1,67 kg/s 210°C 50 bar    5c    280°C    13    11    SW    25    24

H₂O  0,01
CH₄  0,1351
CO   0,0893
CO₂  0,1007
H₂   0,6748

5b    23    16'    1'    15'    2    6

14    H₂O  0,5866

PG 94,6% CH₄    19    7    17    18    8    10    1,67 kg/s; 315°C; 47 bar    CH₄  0,3913
20    260°C    CO   0,000
CO₂  0,0035
H₂   0,0189

9    4    4

1,89 kg/s;  20°C W

0241902

FIG. 3

FIG. 4

FIG. 5

FIG. 6

5/5   0241902

FIG. 7

FIG. 8

Europäisches
Patentamt

# EUROPÄISCHER RECHERCHENBERICHT

| | EINSCHLÄGIGE DOKUMENTE | | | EP 87105491.2 |
|---|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) | |
| X | <u>DE - A1 - 2 834 589</u> (THYSSENGAS GMBH)<br><br>  * Ansprüche 1,10-12; Seite 5, letzter Absatz; Seite 6, letzter Absatz; Seite 7, erster Absatz; Seite 8, Absätze 3, 4; Seite 10, zweiter Absatz; Fig. *<br><br>-- | 1,8,9 | C 07 C 9/04<br><br>C 07 C 1/02 | |
| A | <u>DE - A1 - 2 705 141</u> (LINDE AG)<br><br>  * Seite 11, Zeilen 7-23; Fig. 2; Ansprüche 1,7,8,15 *<br><br>-- | 1,2 | | |
| A | <u>US - A - 4 431 751</u> (HÖHLEIN et al.)<br><br>  * Zusammenfassung *<br><br>---- | 1 | | |
| | | | RECHERCHIERTE SACHGEBIETE (Int. Cl 4) | |
| | | | C 07 C 9/00<br><br>C 07 C 1/00 | |

Der vorliegende Recherchenbericht wurde für alle Patentansprüche erstellt.

| Recherchenort | Abschlußdatum der Recherche | Prüfer |
|---|---|---|
| WIEN | 10-07-1987 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82